# EUROPEAN PATENT APPLICATION

(11) **EP 4 566 447 A2**
(43) Date of publication of application: **11.06.2025**
(21) Application number: 25158618.6
(22) Date of filing: 02.03.2018
(51) Int. Cl.: A01N 1/10, A01N 1/142, A61M 5/50, C12M 1/00, A61M 5/315, A61M 5/32

(54) **DEVICES, METHODS, AND COMPOSITIONS USEFUL IN CRYOPRESERVATION, -STORAGE, -TRANSPORT, AND APPLICATION OF THERAPEUTIC MAMMALIAN CELLS**

(30) Priority: 02.03.2017 US 201762466228 P
(62) Divisional of application: 18760873.2
(71) Applicant: DiscGenics, Inc., Salt Lake City, UT 84116 (US)
(72) Inventor: SILVERMAN, Lara, Salt Lake City UT, 84109 (US); DULATOV, Galina, Salt Lake City UT, 84107 (US); TANDESKI, Terry, Sandy UT, 84093 (US); ERICKSON, Issac, Layton UT, 94040 (US)
(74) Representative: Marks & Clerk LLP

(57) **Abstract**

The disclosed compositions, methods, and devices are useful in freezing, storing, and administering therapeutic eukaryotic cells to a patient in need thereof. In many embodiments, the disclosed therapeutic cells are mammalian cells derived from human intervertebral disc tissue. The disclosed compositions, methods, and devices are able to maintain high viability and sterility of the therapeutic cells, and allow the cells to be administered to a patient directly from the freezing container without manipulation of the cells or transferring to a second container.

## Description

### CROSS-REFERENCE TO RELATED APPLICATIONS

This application claims benefit of priority pursuant to 35 U.S.C. § 119(e) of U.S. provisional patent application No. 62/466,228 entitled "Devices, Methods, And Compositions Useful In Cryo-Preservation Mammalian Cells," filed on March 2, 2017, which is hereby incorporated by reference in its entirety.

### FIELD

The disclosed processes, methods, and systems are directed to preserving therapeutic mammalian cells at very low temperatures for prolonged periods, without significant loss of viability, and may be administered to a patient with little or no processing, after thawing.

### BACKGROUND

Therapeutic cells may be obtained directly from mammalian donors, or may be selected, enriched, amplified, etc. in cell culture before being administered to a patient. In some cases, therapeutic cells may be stored or transported prior to administration. In order to maintain viability of the cells during storage and/or transportation, therapeutic cells may be frozen at very low temperatures to preserve and protect their therapeutic capabilities (cryo-preserved or cryo-storage). This cryo-preservation may enable long-term storage and or extended transportation times with little or no loss in therapeutic efficacy.

Traditional cryo-preservation methods may result in inconsistent or poor viability of therapeutic cells after they are thawed. In addition, traditional methods of cryopreservation require additional processing and manipulation in order to transfer the cells to an application apparatus or device, and/or to remove one or more of the cryopreservation materials.

Consistency, viability, and efficacy may be adversely affected by subsequent manipulation of therapeutic cells. Moreover, transferring therapeutic cells to the appropriate medical device or apparatus increases the risk of the cells being contaminated by unwanted bacteria, viruses, chemicals, etc.. In addition, traditional freezing methods that rely on the use of animal serum may subject human recipients to animal borne diseases.

Thus, improved compositions and methods for use in freezing therapeutic cells, as well as devices that limit manipulation after thawing would be beneficial.

### SUMMARY

Disclosed herein is a composition for cryo storage and preservation of eukaryotic cells in a therapeutic, pharmaceutical composition. The composition includes a freezing media, a freezing agent (e.g. dimethyl sulfoxide), and one or more organic polymers (e.g. hyaluronic acid). The present compositions also allows for freezing the therapeutic cells with and without an animal serum. The disclosed compositions and devices are able to maintain the cells' viability and sterility through freezing, storage at between -130 °C and -196 °C for extended periods of time, thawing, and administration of the cells to a patient.

Also disclosed are containers useful in freezing and thawing eukaryotic cells. In many embodiments, the containers are suitable for administration of the cells after thawing, and require little or no manipulation of the cell mixture prior to administration. In many embodiments, the container may define at least a part of a medical device, such as a syringe, wherein the container may maintain the sterility of the cell mixture during freezing and thawing and may be used to administer the mixture to a patient in need thereof. The container may comprise a lumen, and a first and second open end that may be configured to accept a cap, tip, plug, plunger, or other suitable structure for containing the mixture within the lumen. The container is made of a biocompatible material that maintains its integrity through the freezing and thawing process.

Disclosed herein are devices, compositions, and methods for use in maintaining the viability of therapeutic mammalian cells at low temperatures, the disclosed devices are also useful in reducing manipulation of cells prior to administration to a patient in need thereof. The disclosed compositions, devices, and methods maintain the cells' viability to help reduce loss of therapeutic effect.

Additional objects and advantages of the invention will be set forth, in part, in the description which follows and, in part, will be obvious from the description, or may be learned by practice of the invention. The objects and advantages of the invention will be realized and attained by means of the elements and combinations, particularly pointed out in the appended claims.

### BRIEF DESCRIPTION OF THE DRAWINGS

FIG. 1 shows one embodiment of a device for freezing, storing, and applying the disclosed cell compositions. The upper diagram shows the various parts of the device, unassembled. The lower portion of the figure shows the device in cross-sectional view.
FIG. 2. is a picture of an embodiment of the disclosed device assembled and configured to reduce contamination of the disclosed composition stored within the barrel lumen during freezing, storage, transportation, and thawing.
FIG. 3 compares the recovery and viability between various media, with 1% hyaluronic acid, after 1 month in vapor phase of liquid nitrogen. The freezing container used in these experiments was a syringe system. All media result in good recovery and viability.
FIG. 4 is a bar graph showing a summary of the non-destructive headspace oxygen measurements of the DiscGenics syringe samples. Depicted values are the change in the headspace oxygen concentration between the T0 and T1 timepoints; the headspace oxygen concentration at both timepoints was treated as the average value of 5 repeated measurements of each syringe. The horizontal red line denotes the 3.0% decrease in oxygen that was used as a criterion for failure in the container closure integrity of the syringe.

### DETAILED DESCRIPTION

Provided herein are compositions and methods of freezing eukaryotic cells (e.g., mammalian cells), at very low temperatures, for storage and/or transport. Also provided, are medical devices and apparatuses, useful in containing the frozen cells, that also reduce subsequent manipulation of the cells prior to administration. Methods of freezing and storing the described compositions in the disclosed devices are also described, wherein the methods of freezing and storage provide for surprisingly enhanced resistance to contamination by gases, fluids, and microbes (for example, bacteria, fungi, viruses, etc.).

In many embodiments, the disclosed methods comprise combining the cells with a freezing medium, to create a cell mixture, and then placing the cell mixture in the disclosed devices and apparatuses. In many embodiments, the disclosed freezing compositions comprise a freezing media, a freezing agent, and an organic polymer. In many embodiments, the cells are placed in a container, prior to freezing, wherein the container is part of a medical device useful in administering the cells. In these embodiments, the container is configured to withstand very low temperatures and thawing without substantially affecting the cells' viability and sterility, or the device's functioning.

The present disclosure provides compositions, devices, and methods for freezing and thawing cells in amounts suitable for therapeutic use after thawing. After thawing the disclosed therapeutic cells are 50%-100% viable (i.e. 60-100% of the cells are able to grow and divide), sterile, and ready for immediate administration. In many embodiments, about 50-100% of the cells are recovered from the container and administered to the patient. In many embodiments, the disclosed methods aid in substantially reducing the rate of failure of closure integrity to less than 20%, 10%, 5%, or less. Surprisingly, Applicants have discovered that polymeric syringe plungers, alone, are not sufficient for protecting the sterility of the disclosed compositions, because at very low temperatures, such as those disclosed for freezing and storage of the disclosed cells, the plungers allow for gas exchange that may lead to contamination by biological materials such as viruses, fungi, bacteria, and other microbes.

In many embodiments, the disclosed compositions, methods, and devices help to maintain the potency of the disclosed therapeutic cells, which may be analyzed by measuring expression of one or more indicator genes or proteins, such as production of one or more of a cytokine, cell cycle marker, or extracellular matrix component.

The present disclosure is also useful in freezing a cells at high densities (e.g., approximately 1.0×10⁶ cells/ml to 5.0×10⁶ cells/ml), with or without an animal serum, while maintaining viability, identity, and dispersion of the cells within the composition. In some embodiments the cell density may be greater than about 100x10³, 0.5x10⁶, 1x10⁶, 1.5x10⁶, 2x10⁶, 3x10⁶, 4x10⁶, 5x10⁶, 6x10⁶, 10x10⁶, 20x10⁶, 30x10⁶, 40x10⁶, 50x10⁶, 100x10⁶, 200x10⁶, 300x10⁶, 400x10⁶, 500x10⁶, 600x10⁶, or 700x10⁶, and less than about 750x10⁶, 700x10⁶, 600x10⁶, 500x10⁶, 400x10⁶, 300x10⁶, 200x10⁶, 100x10⁶, 50x10⁶, 40x10⁶, 30x10⁶, 20x10⁶, 10x10⁶, 6x10⁶, 5x10⁶, 4 x10⁶, 3x10⁶, 2x10⁶, 1.5x10⁶, 1x10⁶, 0.5x10⁶, or 200x10³.

In most cases, the therapeutic cells may be frozen at cell densities between 0.5×10⁶ cells/ml and 1.0×10⁷ cells/ml. In some embodiments, the density of therapeutic cells in the cell mixture is between about 1.0x10⁶ and 9.0x10⁶ cells/ml, for example greater than about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 4.0, 4.5, 5.0, 5.5, 6.0, 6.5, 7.0, 7.5, 8.0, and 8.5x10⁶ cells/ml and less than about 9.0, 8.5, 8.0, 7.5, 7.0, 6.5, 6.0, 5.5, 5.0, 4.5, 4.0, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.4, 1.3, 1.2, and 1.1x10⁶ cells/ml.

Cells may be frozen in one or multi dose volumes or amounts. In some embodiments, a dose may be between about 0.1 ml and 5.0 ml, for example greater than about 0.1ml, 0.2 ml, 0.3 ml, 0.4 ml, 0.5 ml, 0.6 ml, 0.7 ml, 0.8 ml, 0.9 ml, 1.0 ml, 1.1 ml, 1.2 ml, 1.3 ml, 1.4 ml, 1.5 ml, 1.6 ml, 1.7 ml, 1.8 ml, 1.9 ml, 2.0 ml, 2.1 ml, 2.2 ml, 2.3 ml, 2.4 ml, 2.5 ml, 2.6 ml, 2.7 ml, 2.8 ml, 2.9 ml, 3.0 ml, 3.1 ml, 3.2 ml, 3.3 ml, 3.4 ml, 3.5 ml, 3.6 ml, 3.7 ml, 3.8 ml, 3.9 ml, 4.0 ml, or 4.5 ml per dose, and less than about 5.0 ml, 4.5 ml, 4.0 ml, 3.9 ml, 3.8 ml, 3.7 ml, 3.6 ml, 3.5 ml, 3.4 ml, 3.3 ml, 3.2 ml, 3.1 ml, 3.0 ml, 2.9 ml, 2.8 ml, 2.7 ml, 2.6 ml, 2.5 ml, 2.4 ml, 2.3 ml, 2.2 ml, 2.1 ml, 2.0 ml, 1.9 ml, 1.8 ml, 1.7 ml, 1.6 ml, 1.5 ml, 1.4 ml, 1.3 ml, 1.2 ml, 1.1 ml, 1.0 ml, 0.9 ml, 0.8 ml, 0.7 ml, 0.6 ml, 0.5 ml, 0.4 ml, 0.3 ml, or 0.2 ml per dose. In some embodiments, 1, 2, 3, or more doses may be frozen in one device.

### Cells

Cells for use with the disclosed compositions, devices, and methods include mammalian cells, therapeutic cells, for example therapeutic human cells. The disclosed cells may be obtained from, or be administered to a various tissues. In many embodiments, therapeutic cells may be obtained from intervertebral disc tissues, and may be administered to intervertebral discs. In other embodiments, various types of cells, for example myoblasts, myocytes, chondrocytes, epithelial, osteocytes, osteoclasts, progenitor cells, stem cells, etc., may be obtained from and/or administered to a variety of tissues, including muscle, liver, heart, lung, pancreas, articular cartilage, tendon, ligament, intervertebral disc, bone, thymus, thyroid, or lymph node. In some embodiments, the disclosed cells may be derived from articular cartilage, heart tissue, or bone.

The disclosed compositions, devices, and methods are useful in treating a variety of diseases and conditions, including without limitation, degenerative disc disease, damaged discs, burns, lacerations, heart and muscle damage, bone fractures/separations, etc.

### Composition

In some embodiments, therapeutic cells are collected from a cell culture media, prior to freezing. In one embodiment, centrifugation is used to collect and pellet the cells. The pelleted cells may then be re-suspended in the disclosed freezing composition to create a cell mixture. In many embodiments, the disclosed freezing composition may comprise a freezing media, a freezing agent, and an organic polymer.

### Media

The freeze media (or cryoprotective media) may comprise one or more additives including, but not limited to, animal serum (e.g. fetal calf serum (FCS) or fetal bovine serum (FBS)) and cryoprotectants (i.e., agents with high water solubility and low toxicity). Cryoprotectants introduced to the freeze media may enhance the survival of cells by limiting or preventing cell damage during the freezing and the thawing processes.

Various commercial freeze media may be used to create the present compositions, for use with the disclosed methods. For example, in some embodiment, animal serum may not be used, in these embodiments a "xeno-free" medium may be used to replace a medium with animal serum, for example Profreeze, FreezlS, Cryostor, etc. In various embodiments, the disclosed freeze media may further comprise one or more additional components, for example proteins such as albumin. In one embodiment, human, mammalian, or synthetic serum albumin (HSA) may be included in the freeze media. In many embodiments, the concentration of freeze media may be the remainder of the fraction not including the freeze agent and organic polymer. In some embodiments, the concentration of freeze media is between about 60% to about 98.9%, for example greater than about 65%, 70%, 75%, 80%, 85%, 86%, 87%, 88%, 89%, 90%, 91%, 92%, 93%, 94%, 95%, 96%, 97%, 98%, or 99% and less than about 100%, 99%, 98%, 97%, 96%, 95%, 94%, 93%, 92%, 91%, 90%, 8%, 88%, 87%, 86%, 85%, 84%, 83%, 82%, 81%, 80%, 75%, or 70%. In one embodiment, the concentration of freeze media is about 91.5%.

The disclosed media may further comprise additional components such as amino acids (e.g., glutamine, arginine, or asparagine), vitamins (including but not limited to B vitamins such as any one or more of vitamin B1, vitamin B2, vitamin B3, vitamin B6, vitamin B7, vitamin B9, or vitamin B12), transition metals (including but not limited to nickel, iron (e.g., ferric iron or ferrous iron), or zinc), and other media components. Any media provided herein may also be supplemented as necessary with hormones and/or other growth factors (such as insulin, transferrin, or epidermal growth factor), ions (such as sodium, chloride, calcium, magnesium, and phosphate), buffers (such as HEPES), nucleosides (such as adenosine and thymidine), trace elements (defined as inorganic compounds usually present at final concentrations in the micromolar range), and glucose or an equivalent energy source. In some aspects, a freezing medium provided herein contains proteins derived from a plant or an animal. In some embodiments, a freezing medium provided herein is free of proteins derived from a plant or an animal. Any other necessary supplements may also be included at appropriate concentrations that would be known to those skilled in the art. Agent

Freezing agents (or cryoprotective agents) may also be referred to as cryoprotective agents. These agents may be chemicals that help the cell maintain viability during the freezing and thawing process. Freezing agents generally enter the cell and aid in reducing intracellular osmotic pressure and protecting proteins in the cell from denaturation. One example of a freezing agent that may cross the cell membrane is DMSO (dimethyl sulfoxide). DMSO's ability to traverse the cell membrane also allows it to leave the cell after thawing. Other freezing agents may include glycerol, alginate, poly vinyl alcohol, etc.

The concentration of freezing agent used may vary from about 1% (by volume or weight) to about 20%. In most embodiments, the concentration of freezing agent may be about 7.5%, for example where the freezing agent is DMSO. In many embodiments, the concentration of DMSO used may be reduced to minimize its administration to the patient being treated with the therapeutic cells.

### Polymers

Organic polymers are included in the freezing composition. The disclosed organic polymers are useful in protecting the cells during freezing and acting as a matrix for the therapeutic cells if they are administered to a patient after thawing. In many embodiments, the organic polymer is a glycosamino glycan, for example hyaluronic acid, sodium hyaluronate, or hyaluronan. In many embodiments the concentration of organic polymer is between about 0.1% and 10% by weight of the mixture. In a preferred embodiment, the concentration of organic polymer is about 1.0%.

### Methods

The disclosed cell mixtures may be placed within a suitable container that is designed to withstand very low temperatures (i.e. less than about -100 °C). In some embodiments, the freezing composition may be chilled prior to combining with the therapeutic cells. In many embodiments, the cells may be frozen at -20°C to -80 °C for between 5 and 120 min. before placing in long-term storage. In some embodiments, the compositions and devices may be stored at about -80 °C or about -196 °C for long-term storage. For example the disclosed methods may including freezing cells in disclosed devices at about -20°C, -25 °C, -30°C, -35 °C, -40 °C, -45 °C, -50°C, -55 °C, -60 °C, -65 °C, -70 °C, -75 °C, or -80 °C for greater than about 5 min., 10 min., 15 min., 20 min., 25 min., 30 min., 35 min., 40 min., 45 min., 60 min., 75 min., 90 min., 105 min., 120 min., or 180 min., and less than about 240 min., 180 min., 120 min., 105 min., 90 min., 75 min., 60 min., 45 min., 40 min., 35 min., 30 min., 25 min., 20 min., or 15 min. In many cases, the temperature of the composition may be decreased at rate greater than about 0.1° C/min, 0.2° C/min, 0.3° C/min, 0.4° C/min, 0.5° C/min, 1° C/min, 2° C/min, 3° C/min, 4° C/min, 5° C/min, 6° C/min, 7° C/min, 8° C/min, 9° C/min, or 10° C/min, and less than about 15° C/min, 10° C/min, 9° C/min, 8° C/min, 7° C/min, 6° C/min, 5° C/min, 4° C/min, 3° C/min, 2° C/min, 1° C/min, 0.5° C/min, 0.4° C/min, 0.3° C/min, 0.2° C/min, or 0.1° C/min.

The disclosed methods may involve a one-step, multi-step, or ramped freezing. In most embodiments, after the cells are frozen, they may be stored in liquid nitrogen vapor phase, which may be from about -135 °C to about -196 °C, for example less than about -130 °C, -135 °C, -140 °C, -145 °C, -150 °C, -155 °C, -160 °C, -165 °C, -170 °C, -171 °C, -172 °C, -173 °C, -174 °C, -175 °C, -176 °C, -177 °C, -178 °C, -179 °C, -180 °C, -181 °C, -182 °C, - 183 °C, -184 °C, -185 °C, -186 °C, -187 °C, -188 °C, -189 °C, -190 °C, -191 °C, -192 °C, - 193 °C, -194 °C, -195 °C, or -196 °C, and greater than about 196 °C, -197 °C, -196 °C, -195 °C, -194 °C, -193 °C, -192 °C, -191 °C, -190 °C, -189 °C, -188 °C, -187 °C, -186 °C, -185 °C, -184 °C, -183 °C, -182 °C, -181 °C, -180 °C, -179 °C, -178 °C, -177 °C, -176 °C, -175 °C, - 174 °C, -173 °C, -172 °C, -171 °C, -170 °C, -165 °C, -160 °C, -155 °C, -150 °C, -145 °C, - 140 °C, and -135 °C.

The disclosed cell compositions may be stored for long periods of time. In some embodiments, the cells may be stored for greater than about 4 weeks, 6 weeks, 8 weeks,10 weeks, 12 weeks, 16 weeks, 20 weeks, 24 weeks, 28 weeks, 32 weeks, 36 weeks, 40 weeks, 44 weeks, 48 weeks, 52 weeks, 1 year, 1.5 years, 2 years, 2.5 years, 3 years, 3.5 years, 4 years, or more, and less than about 5 years, 4.5 years, 4 years, 3.5 years, 3 years, 2.5 years, 2 years, 1.5 years, 1, year, 52 weeks, 48 weeks, 44 weeks, 40 weeks, 36 weeks, 32 weeks, 28 weeks, 24 weeks, 20 weeks, 16 weeks, 12 weeks, 10 weeks, 8 weeks, or 6 weeks. In most embodiments, viability of stored cells is greater than about 90% after thawing, for example less than about 100%, 99%, 98%, 97%, 96%, 95%, 94%, 93%, 92%, 91%, 90%, 89%, 88%, 87%, 86%, 85%, 80%, and 75%, and greater than about 75%, 80%, 85%, 86%, 87%, 88%, 89%, 90%, 91%, 92%, 93%, 94%, 95%, 96%, 97%, 98%, or 99%.

Cells, compositions, and devices may be shipped at various temperatures. In some embodiments frozen cells may be shipped in various media including liquid nitrogen, vapor phase nitrogen. In other embodiments, the disclosed methods may include prolonged storage and shipping at temperatures from -80°C to -196 °C.

Prior to freezing, the cells are resuspended in the freezing composition and deposited in a container, under conditions that maintain the mixture's sterility, composition, and prevent or inhibit voids or spaces between the mixture and the container. In some embodiments, the device may be preloaded with part of the composition and the cells may be added to the composition within the container. In many embodiments, the container is at least part of a medical device, for example a syringe barrel, but may also be any container, suitable for maintaining the viability of the cells at low temperature. In many embodiments, the container may aid in directly administering or applying the therapeutic cells to a patient. In some embodiments, the container may be implantable and/or biodegradable. In some embodiments, as described in more detail below, the medical device may be a container, such as a syringe barrel that may be capped or sealed to prevent gas exchange between the composition and nitrogen vapor, or other gases, surrounding the device.

The disclosed methods may be useful in increasing the rate at which the temperature of cells is reduced. More rapid reduction of temperature may help to maintain cell viability by avoiding some damaging aspects of freezing (for example dehydration). Disclosed thaw rates may also be faster than traditional methods. In most embodiments, thawing may be performed by placing the container at room or ambient temperature (e.g. about 20 °C to about 25 °C), and allowing the cell mixture to achieve a liquid form. In other embodiments, thawing may be performed on ice, or with the assistance of a warming device. In some embodiments, the cell mixture may be allowed to achieve a liquid form, that is greater than about 0 °C in less than about 2 hrs, 110 min., 100 min., 95 min., 90 min., 85 min., 80 min., 75 min., 70 min., 65 min., 60 min., 55 min., 50 min., 45 min., 40 min., 35 min., 30 min., 25 min., or 20 min., and greater than about 15 min., 20 min., 25 min., 30 min., 35 min., 40 min., 45 min., 50 min., 55 min., 60 min., 65 min., 70 min., 75 min., 80 min., 85 min., 90 min., 95 min., 100 min., 105 min., 110 min., or 115 min. In some embodiments, the cell mixture is administered to a patient before the cell mixture is at body temperature (i.e. about 37 °C), that is less than about 37 °C, 36 °C,.35 °C, 34 °C, 33 °C, 32 °C, 31 °C, 30 °C, 29 °C, 28 °C, 27 °C, 26 °C, 25 °C, or 20 °C, and greater than about 15 °C, 20 °C, 25 °C, 26 °C, 27 °C, 28 °C, 29 °C, 30 °C, 31 °C, 32 °C, 33°C, 34 °C, 35 °C, or 36 °C.

### Medical Device

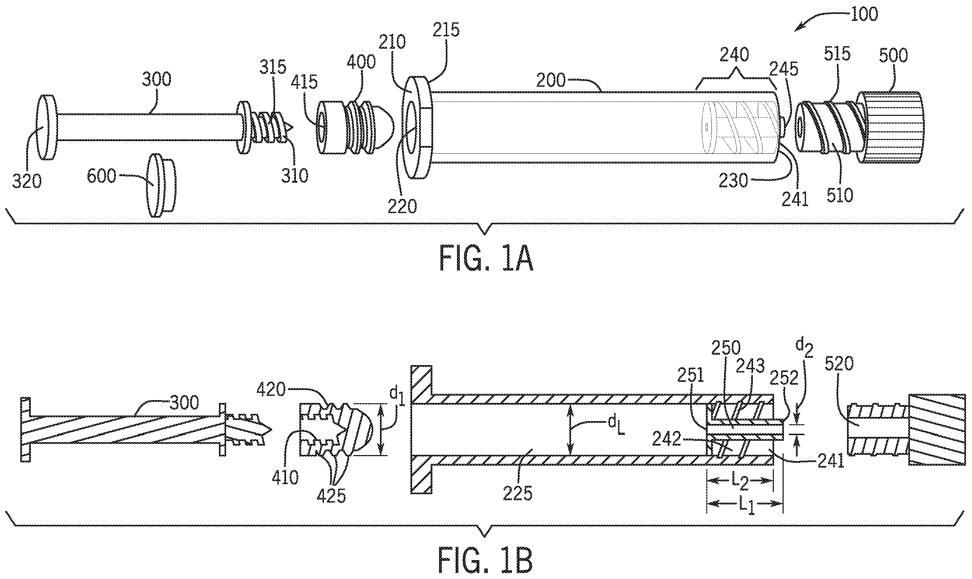

The cells may be transferred to a medical device or container prior to freezing. In some embodiments, the medical device may be the barrel or lumen of a syringe, and the composition may be sealed within the barrel with one or more seals, plungers, caps, or other suitable barriers against gas exchange and/or contamination from substances or organisms surrounding the syringe. Cryopreservation, storage, and transfer of the disclosed cells in and from a syringe device may aid in reducing costs associated manufacture, shipping, and application of the disclosed cells. For example, use of vials may increase time and costs due to the necessity of having to transfer cells to a syringe for application. The use of a syringe may also reduce the need to pass the disclosed cells through more than one needle, thus reducing exposure to forces such as shear that may affect potency. Finally, transferring the disclosed cells from a storage container to a applicator may also result in loss of sample that is left in the first container. FIG. 1 shows an embodiment of the described container device, wherein the device is a syringe.

With reference to FIG. 1, the disclosed device 100, may include a syringe barrel 200, a plunger rod 300, a plunger 400, and a needle end cap 500. An optional barrel cap 600 is also shown. In many embodiments, the syringe may not include a plunger rod. The barrel may have a first plunger end 210 comprising a barrel flange 215, and a barrel orifice 220 defining an opening into a barrel lumen 225, and a second needle end 230. The barrel orifice having a diameter d₁, and the lumen having a diameter, D_{L}. The barrel flange end may define a substantially flat surface surrounding the barrel orifice, and having a diameter greater than an outer diameter of the barrel. At or near the needle end may be a needle hub 240 that may define a needle hub lumen 241 defined by an interior surface 242 comprising a plurality of raised cap or luer receiving ridges 243. A tip 245 may be positioned within the center of the needle hub lumen for receiving a needle (not shown) or, optionally, the needle end cap 500. The tip defining a lumen 250 with a first, entrance orifice 251 in fluid communication with the barrel lumen, and a second exit orifice 252 that may aid in transferring compositions within the barrel lumen into a needle (not shown). The second exit orifice having a diameter d₂, that may be selected to minimize shear forces when the cell composition passes through the tip lumen and into a syringe needle (not shown). In various embodiments the tip lumen may have a constant diameter equal to d₂, or a variable diameter wherein the first entrance orifice defines a diameter that is greater than d₂, but less than d₁. The tip lumen has an internal diameter that is smaller than the internal diameter of the barrel lumen D_{L}, which may be between about 1.0 to 10.0 mm. In many embodiments, the barrel lumen has an internal diameter of about 6.0, 6.1, 6.2, 6.3, or 6.4 mm, and the tip lumen may be greater than about 0.5 mm, 0.6 mm, 0. 7 mm, 0.8 mm, 0.9 mm, 1.0 mm, 1.1 mm, 1.2 mm, 1.3 mm, 1.4 mm, 1.5 mm, 1.6 mm, 1.7 mm, 1.8 mm, 1.9 mm, 2.0 mm, 2.1 mm, 2.2 mm, 2.3 mm, or 2.4 mm, and less than about 2.5 mm, 2.4 mm, 2.3 mm, 2.2 mm, 2.1 mm, 2.0 mm, 1.9 mm, 1.8 mm, 1.7 mm, 1.6 mm, 1.5 mm, 1.4 mm, 1.3 mm, 1.2 mm, 1.1 mm, 0.9 mm, 0.8 mm, 0.7 mm, or 0.6 mm. The tip may further define a length L₁ that is greater or less than the depth of the needle hub lumen L₂, for example less or greater than about 0.1 mm, 0.2 mm, 0.3 mm, 0.4 mm, 0.5 mm, 0.6 mm, 0.7 mm, 0.8 mm, 0.9 mm, 1.0 mm, 1.1 mm, 1.2 mm, 1.3 mm, 1.4 mm, 1.5 mm, 1.6 mm, 1.7 mm, 1.8 mm, 1.9 mm, 2.0 mm, 2.1 mm, 2.2 mm, 2.3 mm, or 2.4 mm. Thus, the tip may protrude from the needle hub lumen (if the length is greater than the depth) or may be recessed (if the length is less than the depth). Some embodiments of the disclosed syringe may include a needle end cap, or needle while others do not. The needle end cap 500 may be configured to securedly seal the tip and prevent gas exchange, cell composition leakage from the barrel lumen, and/or ingress of gas, microbes, or other foreign material into the barrel. In the embodiment shown in FIG. 1, the needle end cap may have an outer surface 510 defining a plurality of connector ridges 515 for engaging complementary structures on the interior surface of the needle hub lumen. In the embodiment shown in FIG. 1, the needle end cap may further define an interior tip lumen 520 for accepting a tip on the barrel.

The plunger rod may be connected to the plunger by various methods. In an embodiment, such as is shown in FIG. 1, the plunger rod may include a plunger connector 310 at one end and a plunger head 320 at the opposite end. The plunger connector may be configured to fixedly connect to the plunger. In the embodiment shown in FIG. 1, the plunger connector defines a screw with a plurality of screw tines 315. The plunger may define a plunger acceptor structure 415 for fixedly connecting with the plunger connector on the plunger rod. The embodiment shown in FIG. 1 has a plunger acceptor configured as a lumen for accepting the plunger connector screw of the plunger rod. The plunger may have an outside surface 420 configured to contact the inner lumen of the barrel. In the embodiment of FIG. 1, the plunger includes a plurality of ridges 425 on the outside surface.

Applicants have surprisingly discovered that polymeric are not able to maintain a gas tight seal within the barrel of a syringe when the syringe is stored at temperatures below about -80 °C. In many cases, the failure of the polymeric plunger is not affected by removal of the plunger rod from the plunger. That is, even applications where a plunger is fixedly connected to a plunger rod showed incomplete closure. Applicants have also surprisingly shown that the use of a polymeric cap at or near the flange orifice of the barrel may help to prevent gas exchange and/or contamination. As shown in Example 2, below, gas exchange/leakage is not prevented by additional plungers being placed in the barrel. Rather, sealing the flange end of the barrel is able to significantly reduce the rate of failure. Other methods for sealing the flange end of the syringe may include placing other suitable polymeric and non-polymeric seals at or near the flange end of the barrel. In one embodiment, an adhesive may be positioned between the polymeric cap and the flat surface of the flange to aid in sealing, in other embodiments the cap may be sealed to the flat surface using other techniques known to those of skill in the art.

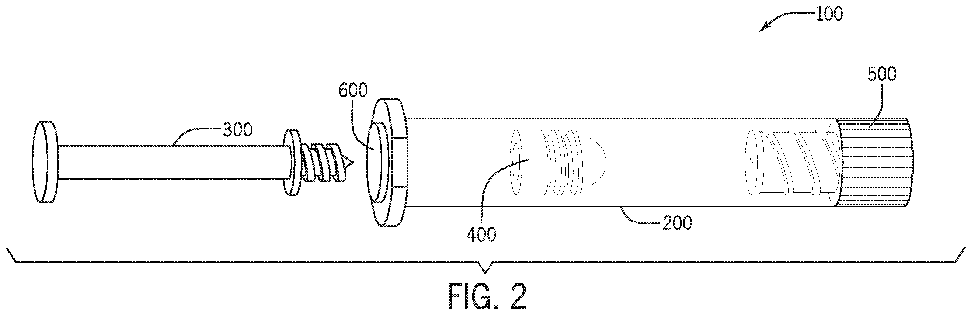

FIG. 2 shows one embodiment of the disclosed device, configured to maintain closure integrity and reduce contamination of the composition by fluid, gas, microbes, or other materials. In this embodiment, the barrel orifice is sealed by placing the barrel cap 600 at least partially within the barrel end orifice. In some embodiments, the cap may be held in place by one or more structures or techniques to prevent gas exchange and/or contaminants from entering the barrel lumen. The barrel cap may be manufactured of any compound or element that may seal to the barrel flange and/or barrel orifice to prevent contamination of the barrel lumen. In some embodiments, additional structures may help secure the barrel cap, for example ridges (such as those seen in the needle end cap), polymeric adhesives, metal closures, etc.

The disclosed plungers, needle end caps, and barrel caps may be manufactured from various polymeric and non-polymeric materials suitable for use. In some embodiments, the structures may be made from silicone, polypropylene, butyl rubber, natural rubber or other suitable material. In many embodiments, the barrel lumen, plunger, needle end cap, and/or barrel cap may further include a coating, layer, or application of a substance to aid in sealing and reducing friction. In some embodiments, for example, the coating may be silicone, **PTFE** (polytetrafluoroethylene) or other suitable material for use in medical applications that are well known in the art.

The disclosed medical devices, may aid in freezing, storing, thawing, and administering therapeutic cells. In most embodiments, the disclosed devices include a body having an open first end and an open second end, and a lumen disposed between and fluidly connecting the openings. The first open end may define a tip configured to removably attach and detach a sealing cap or an applicator, for example a needle. The tip may define an interior in fluid communication with the lumen, and an exterior. The exterior of the tip may include one or more structures designed to aid in securing the needle, for example a luer lock. In other embodiments, the cap and needle may be pressure fitted.

The second open end may define an opening configured to accept the disclosed therapeutic cells in a freezing composition so that they may be deposited within the lumen. The second opening may be further configured to accept a cap, plunger, plug, or other structure designed to fit within and movably seal to walls of the second opening. The walls of the second opening may be contiguous with walls of the lumen. The cap, plunger, plug, or other structure may be designed to connect (in some embodiments reversibly) rod portion that may aid in transferring a force to the cap, plunger, plug, or other structure, that may cause the cap, plunger, plug, or other structure to move relative the walls of the lumen.

The disclosed devices are designed to maintain function after being stored at very low temperatures and in contact with one or more components of a freezing composition. For example, the disclosed cap, plunger, plug, or other structure may substantially maintain a fluid seal with the walls of the second open end and/or lumen during freezing, storage, and thawing, to aid in preventing contamination of therapeutic cells positioned within the lumen.

The walls of the second open end and the lumen are designed to allow the cap, plunger, plug, or other structure to move smoothly against it while substantially maintaining a fluid seal. In some embodiments, the lumen may comprise a coating on the walls that may prevent chemical interaction between the device and the freezing composition. In other embodiments, the composition of the lumen wall may be sufficient to prevent chemical interaction. Chemical interaction may refer to a reaction that may change either the composition of the device or the freezing composition that may decrease or alter the therapeutic effect of the cells and/or freezing composition.

The disclosed devices may be manufactured from various materials. In some embodiments, the materials used to manufacture the disclosed device resists excessive expansion, contraction, breakage, cracking, shattering, etc. when frozen and thawed. In one embodiment, the device may comprise a polymeric glass substitute, for example a polymeric cyclic olefin such as crystal zenith, or cyclo olefin polymer and copolymers. In many embodiments, the disclosed device may include one or more transparent materials that may allow the therapeutic cells and freezing composition to be monitored.

The wall of the lumen is sufficiently thin to allow for controlled and rapid freezing and thawing. In many embodiments, the material of the device lumen may efficiently conduct heat to allow for rapidly freezing and thawing of the therapeutic cells.

The cap, plug, plunger, or other materials may be at least partially manufactured of a polymeric compound that resists expansion, contraction, cracking, shattering etc. when frozen and thawed. In some embodiments, the polymeric compound comprises one or more organic polymers, such as isoprene or latex. In some embodiments, a lubricant or coating may be added to the cap, plug, plunger, or other materials to aid in maintaining a seal against the lumen wall.

### Definitions

"Medium," "media," and "cell culture medium/media" refer to solutions for maintaining the viability of eukaryotic cells in culture, and in situ (e.g. after administering to a patient in need of such cells). The disclosed medium comprises a nutrient source (e.g. one or more of serum, serum substitute, glucose, galactose, etc.) used for growing cells, and one or more additional components (e.g. hormones, peptides, vitamins, essential amino acids, non-essential amino acids, minerals, salts, buffers, etc).

A "freezing medium," may refer to a medium containing one or more additional components, for example a cryoprotective agent, useful for maintaining viability of a eukaryotic cell during and after freezing.

While multiple embodiments are disclosed, still other embodiments of the present invention will become apparent to those skilled in the art from the following detailed description. As will be apparent, the invention is capable of modifications in various obvious aspects, all without departing from the spirit and scope of the present invention. Accordingly, the detailed description is to be regarded as illustrative in nature and not restrictive.

All references disclosed herein, whether patent or non-patent, are hereby incorporated by reference as if each was included at its citation, in its entirety. In case of conflict between reference and specification, the present specification, including definitions, will control.

Although the present disclosure has been described with a certain degree of particularity, it is understood the disclosure has been made by way of example, and changes in detail or structure may be made without departing from the spirit of the disclosure as defined in the appended claims.

### EXAMPLES

### Example 1 - Recovery and Viability in Freezing Composition

Cell recovery and viability in various medium was compared. Cells were frozen and stored in in vapor phase of liquid nitrogen for one month in a container device as disclosed above. The container for these experiments was a syringe system. Storage in the disclosed syringe system allows for direct application of the cell mixture, after thawing, to a subject while minimizing manipulation.

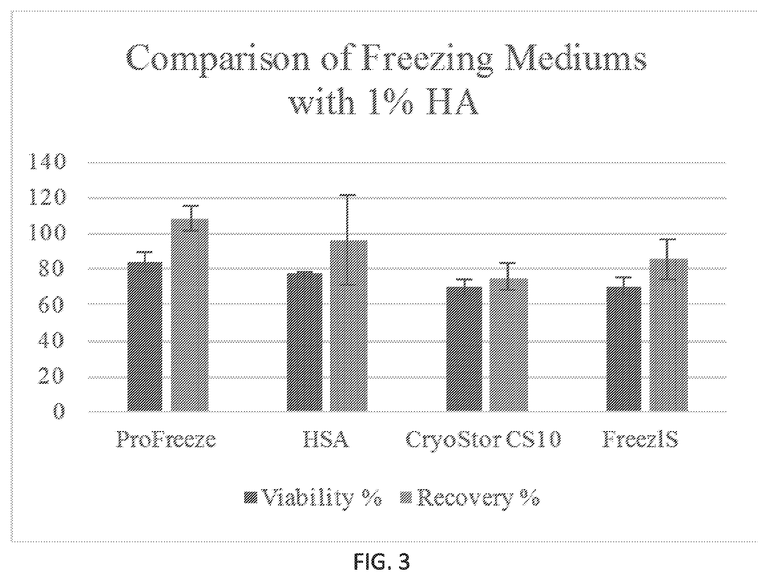

The bar graph in Fig. 3 demonstrates that all tested freezing compositions resulted in good recovery and viability.

### Example 2 - Closure Integrity

Surprisingly, initial studies showed that freezing and thawing of samples in syringes at very low temperatures, such as those in liquid nitrogen vapor, resulted in loss of oxygen in the sample. This indicated that the polymeric plunger and/or tip cap were not sufficient to maintain a seal during freezing, storage, and thawing. This failure was seen both when the plunger rod was in place and when it was removed.

To test the source of this failure a series of configurations were tested using various syringes and closures. The objective of this study was to evaluate the container closure integrity (CCI) of multiple syringe configurations stored at cryogenic temperatures by using non-destructive analysis of the syringe headspace oxygen concentration [%] and its total pressure [torr]. For this study, multiple syringes were prepared representing eight different syringe configurations. The syringes were filed with sample and stored for ~4.5 days in a cryogenic storage container. Under these conditions, a breach in container closure integrity would lead to a decrease in the oxygen concentration within the syringe headspace. This decrease in oxygen concentration would likely result from gas leakage and exchange between the nitrogen-rich storage environment and the composition within the syringe.

As a control, one syringe from each configuration was stored at room temperature in a nitrogen-purged gas-tight container. These negative control samples were designed to investigate the effect of the cryogenic storage temperature on gas exchange/leakage.

Headspace oxygen concentration measurements were conducted by using a tunable diode laser absorption spectroscopy (TDLAS) technique.

### Sample Set

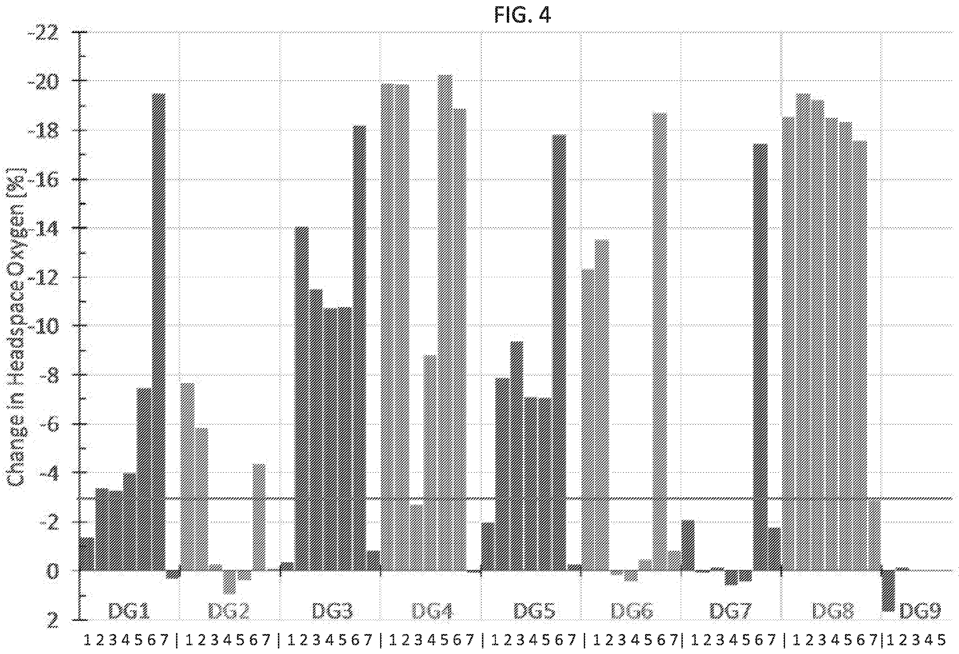

A variety of syringes, plungers, and stoppers were combined to test different configurations (Figure 4 and Table 1) for closure integrity. Seven replicate samples were prepared for the Sample Formats described in Table 1, below.

For each replicate, the oxygen content and total pressure of the headspace of each syringe was measured prior to storage, and then again after storage.

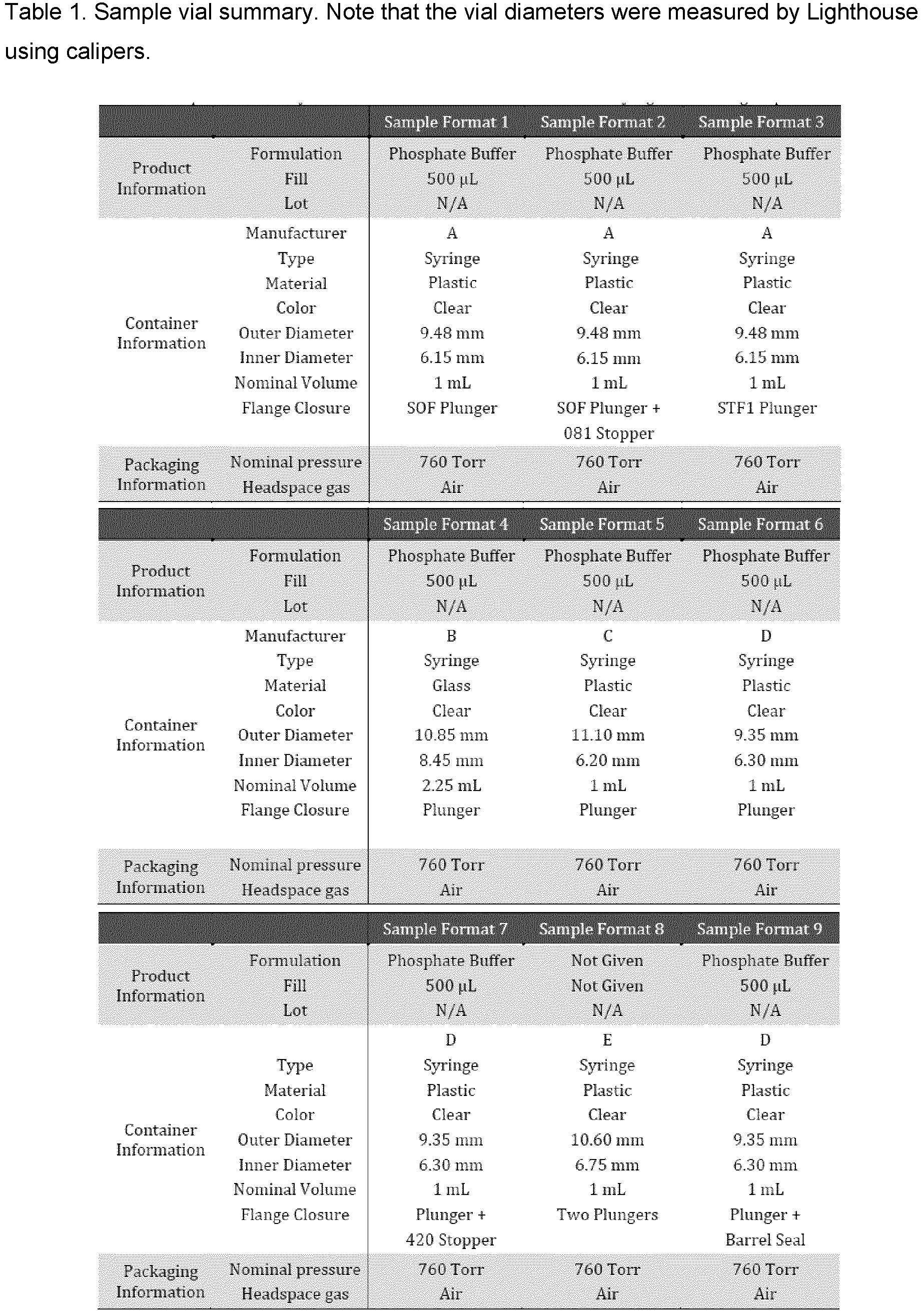

### Method and Materials

### Sample Preparation and Handling

For these experiments, PBS was used as a test composition. A Gilson P1000 Pipetman was used to transfer the PBS into each syringe (Gibco; Cat#: 20012-027; pH 7.2). Care was taken to avoid formation of liquid droplets on the inside surface of the syringe barrel. A Dabrico vented placement tool (Dabrico; Cat#: MS-25) was used to introduce the indicated plunger into the syringe.

For several Sample Formats, an additional barrier was created by inserting a 081 Stopper and a 420 Stopper. Specifically, these stoppers were manually inserted into the syringes for Sample Format 2 and 7, respectively.

Sample Numbers 1 through 5 represented the experimental group for that particular sample format. Sample Number 6 represented a positive control with a known, fabricated defect. The defect for the Sample Number 6 replicates was introduced by inserting a syringe needle through the plunger (and stopper, if present) of the syringe. Specifically, a 23 gauge × 1 inch needle was used for all sample formats except for Sample Format 7, for which an 18 gauge × 1.5 inch needle was used (necessary to pierce through both plunger and stopper). Sample Number 7 of each sample format represented the negative control that was stored at room temperature in a nitrogen environment, as described above.

The initial timepoint (T0) for both headspace oxygen concentration and total pressure was measured on the day that sample formats were fabricated. On day two,

Sample Number 1 through 6 of each of sample format was placed, first in a -20°C freezer for 3 hrs and then transferred to a -80°C freezer. After each sample was held -80° C for ~4 hrs, Sample Numbers 1 through 6 were stored in the Cryoport Express^{®} High Volume Dry Vapor Shipper (all excess liquid nitrogen was removed). The temperature, monitored using an Apollo IV digital thermometer (UEi; Cat#: DT304) for the dry vapor shippers, remained stable at -177°C (96 K) throughout the ~4.5 day cryo-storage period. Again, Sample Numbers 7 were stored in a gas-tight container purged with pure nitrogen and maintained at ambient room temperature (~23°C) for ~4.5 days.

After ~4.5 days of storage, Sample Numbers 1 through 6 were removed from cryo-storage. The samples were immediately placed in a second gas-tight, nitrogen-purged at ambient room temperature (~23°C) for about 2 hr, to allow the samples to thaw and equilibrate to room temperature.

The T1 timepoint measurement for both headspace oxygen concentration and total pressure was measured for all syringes of all formats. Each sample was measured five times. FIG. 4 is a graph of change in oxygen headspace concentration from T0 to T1.

### Headspace Oxygen Measurements

Frequency Modulated Tunable Diode Laser Absorption Spectroscopy instrument (Lighthouse) was used to measure the headspace oxygen in each replicate. This technique, termed TDLAS provides rapid and non-invasive gas analysis of the headspace within sealed containers to provide both specific gas number density and total headspace pressure.

Prior to sample analysis, six oxygen standards with known oxygen concentrations were each measured 5 consecutive times to verify performance of the instrument. Based upon the absolute value of the measurement error (the difference between the known and measured values) and the measurement precision (the standard deviation of multiple measurements for each standard), the headspace oxygen concentration measurement uncertainty was established as ±0.8% atm for this study.

### Headspace Pressure Measurements

Headspace pressure measurements were performed using a FMS-Moisture/Pressure Headspace Analyzer (Lighthouse) following standard measurement procedures. Briefly, the instrument was turned on and allowed to warm up, with a nitrogen purge of at least 1 standard liter/minute, for at least thirty minutes prior to the measurement session. Calibration was then performed with known pressure/moisture standards.

Prior to sample analysis, pressure standards (10 standards for one format, 8 for a second format) at known total pressures were each measured 5 consecutive times to verify performance of the instrument. Based upon the absolute value of the measurement error (the difference between the known and measured value) and the measurement precision (the standard deviation of multiple measurements for each standard), the headspace total pressure measurement uncertainty was established for this study as the greater of ±6 torr and ±7% of the measured value.

### Results

A summary of the non-destructive headspace oxygen measurements is presented in Figure 4. The change in the headspace oxygen concentration between the T0 and T1 timepoints is displayed for each of the syringes in the nine different Sample Formats. The horizontal red line denotes the 3.0% decrease in oxygen that was used as a criterion for failure in the container closure integrity of the syringe (see Discussion below). The headspace oxygen concentration measurement uncertainty for the standards used in this study was established as ±0.8% atm based upon the performance of the oxygen standards. Table 3 summarizes the results of each measured headspace parameter for each syringe sample. The headspace total pressure measurement uncertainty for these sample formats was established as the greater of ~6 torr or ~7% of the measured value based upon the performance of the Lighthouse pressure standards.

**Table 3. Average measured headspace oxygen, total pressure, and moisture values for each DiscGenics syringe sample. Listed values are the average values of 5 repeated measurements of each sample. A 3.0% decrease in oxygen was used as the primary criterion for identifying a failure in the container closure integrity of the syringe (see**

| | T0 | | | T1 | | | | Change | | |
|---|---|---|---|---|---|---|---|---|---|---|
| Sample Label | Moisture [torr] | Total [torr] | Oxygen [%] | Moisture [torr] | Total [torr] | Oxygen [%] | Plunger Failure | Oxygen [%] | Total [torr] | CCI Failure |
| DG1-1 | 26.0 | 792.4 | 20.9 | 13.9 | 788.1 | 19.5 | Yes | -1.4 | -4.4 | Yes |
| DG1-2 | 26.1 | 790.4 | 20.8 | 12.8 | 777.2 | 17.4 | Yes | -3.4 | -13.2 | Yes |
| DG1-3 | 26.1 | 785.4 | 20.7 | 14.5 | 780.4 | 17.4 | Yes | -3.3 | -5.1 | Yes |
| DG1-4 | 26.3 | 791.7 | 20.3 | 26.4 | 790.4 | 16.3 | | -4.0 | -1.3 | Yes |
| DG1-5 | 25.9 | 792.0 | 20.6 | 25.6 | 852.5 | 13.1 | | -7. 5 | 60.5 | Yes |
| DG1-6 | 26.8 | 797.3 | 21.7 | 25.3 | 792.7 | 2.2 | | -19.5 | -4.5 | Yes |
| DG1-7 | 28.5 | 791.4 | 20.4 | 25.8 | 774.0 | 20.7 | | 0.3 | -17.4 | |
| DG2-1 | 25.9 | 844.0 | 21.5 | 25.6 | 1004.8 | 13.7 | | -7.7 | 160.8 | Yes |
| DG2-2 | 26.3 | 844.1 | 20.9 | 25.5 | 1032.5 | 15.0 | | -5.9 | 188.4 | Yes |
| DG2-3 | 25.8 | 839.5 | 21.4 | 26.3 | 831.1 | 21.1 | | -0.3 | -8.3 | |
| DG2-4 | 26.7 | 855.4 | 21.0 | 26.0 | 844.3 | 22.0 | | 0.9 | -11.1 | |
| DG2-5 | 26.8 | 854.5 | 21.1 | 26.1 | 843.6 | 21.5 | | 0.4 | -11.0 | |
| DG2-6 | 26.7 | 849.0 | 20.6 | 26.3 | 794.6 | 16.2 | | -4.4 | -54.3 | Yes |
| DG2-7 | 26.9 | 855.4 | 21.5 | 25.8 | 830.4 | 21.4 | | -0.1 | -25.1 | |
| DG3-1 | 27.3 | 795.2 | 21.5 | 26.8 | 783.7 | 21.1 | | -0.4 | -11.5 | |
| DG3-2 | 26.8 | 794.0 | 21.4 | 25.4 | 1001.8 | 7.3 | | -14.1 | 207.7 | Yes |
| DG3-3 | 26.5 | 786.5 | 20.7 | 25.8 | 1004.1 | 9.1 | | -11.5 | 217.6 | Yes |
| DG3-4 | 26.5 | 780.0 | 20.7 | 26.5 | 1008.8 | 10.0 | | -10.7 | 228.8 | Yes |
| DG3-5 | 25.9 | 788.4 | 21.0 | 25.8 | 1021.4 | 10.2 | | -10.8 | 233.0 | Yes |
| DG3-6 | 26.6 | 785.0 | 20.9 | 25.8 | 787.9 | 2.7 | | -18.2 | 2.9 | Yes |
| DG3-7 | 26.8 | 790.9 | 21.2 | 25.7 | 772.5 | 20.3 | | -0.8 | -18.4 | |
| DG4-1 | 28.3 | 806.0 | 20.3 | 25.1 | 886.6 | 0. 4 | | -19.9 | 80.6 | Yes |
| DG4-2 | 26.7 | 803.2 | 20.4 | 25.3 | 915.5 | 0.5 | | -19.9 | 112.3 | Yes |
| DG4-3 | 26.4 | 807.2 | 20.2 | 21.1 | 783.2 | 17.4 | Yes | -2.7 | -23.9 | Yes |
| DG4-4 | 25.8 | 806.4 | 20.4 | 21.7 | 759.7 | 11.6 | Yes | -8.8 | -46.7 | Yes |
| DG4-5 | 26.5 | 807.7 | 20.5 | 25.0 | 911.2 | 0.2 | | -20.3 | 103.4 | Yes |
| DG4-6 | 27.2 | 809.6 | 20.3 | 23.8 | 840.2 | 1.4 | | -18.9 | 30.6 | Yes |
| DG4-7 | 26.9 | 802.5 | 20.2 | 24.9 | 811.0 | 20.3 | | 0.1 | 8.4 | |
| DG5-1 | 22.8 | 795.9 | 20.5 | 19.3 | 773.7 | 18.5 | Yes | -2.0 | -22.2 | Yes |
| DGS-2 | 23.0 | 797.1 | 20.8 | 19.0 | 766.1 | 12.9 | Yes | -7.9 | -31.0 | Yes |
| DG5-3 | 22.7 | 798.3 | 20.5 | 20.1 | 777.8 | 11.1 | Yes | -9.4 | -20.5 | Yes |
| DG5-4 | 21.5 | 788.6 | 20.6 | 19.0 | 780.9 | 13.4 | Yes | -7.1 | -7.7 | Yes |
| DG5-5 | 22.4 | 799.2 | 20.9 | 17.8 | 771.9 | 13.8 | Yes | -7.1 | -27.3 | Yes |
| DG5-6 | 21.0 | 804.3 | 20.3 | 22.2 | 788.3 | 2.5 | | -17.8 | -16.1 | Yes |
| DG5-7 | 23.9 | 804.1 | 20.9 | 22.4 | 788.9 | 20.7 | | -0.3 | -15.2 | |

### Discussion).

**Table 3 (cont.). Average measured headspace oxygen, total pressure, and moisture values for each DiscGenics syringe sample. Listed values are the average values of 5 repeated measurements of each sample. A 3.0% decrease in oxygen was used as the primary criterion for identifying a failure in the container closure integrity of the syringe (see**

| | T0 | | | T1 | | | | Change | | |
|---|---|---|---|---|---|---|---|---|---|---|
| Sample Label | Moisture [torr] | Total [torr] | Oxygen [%] | Moisture [torr] | Total [torr] | Oxygen [%] | Plunger Failure | Oxygen [%] | Total [torr] | CCI Failure |
| DG6-1 | 26.3 | 796.4 | 20.8 | 27.1 | 907.2 | 8.4 | | -12.4 | 110.8 | Yes |
| DG6-2 | 26.4 | 796.7 | 20.6 | 26.2 | 908.0 | 7.0 | | -13.5 | 111.3 | Yes |
| DG6-3 | 26.2 | 798.5 | 21.3 | 26.9 | 786.6 | 21.4 | | 0.2 | -11.9 | |
| DG6-4 | 26.7 | 794.4 | 20.9 | 25.7 | 785.1 | 21.3 | | 0.5 | -9.3 | |
| DG6-5 | 26.2 | 790.8 | 21.5 | 25.7 | 774.2 | 21.0 | | -0.5 | -16.6 | |
| DG6-6 | 26.1 | 794.0 | 21.2 | 25.2 | 785.8 | 2.5 | | -18.8 | -8.2 | Yes |
| DG6-7 | 26.6 | 796.3 | 21.5 | 25.3 | 774.4 | 20.6 | | -0.9 | -21.9 | |
| DG7-1 | 26.8 | 893.1 | 21.2 | 26.8 | 1093.3 | 19.2 | | -2.1 | 200.2 | Yes |
| DG7-2 | 28.2 | 895.0 | 21.0 | 26.1 | 887.2 | 21.1 | | 0.1 | -7.8 | |
| DG7-3 | 26.3 | 895.0 | 21.6 | 26.0 | 876.9 | 21.5 | | -0.2 | -18.1 | |
| DG7-4 | 26.3 | 891.3 | 21.4 | 26.0 | 877.8 | 22.0 | | 0.6 | -13.5 | |
| DG7-5 | 27.4 | 877.1 | 21.3 | 25.4 | 860.4 | 21.8 | | 0.5 | -16.7 | |
| DG7-6 | 26.4 | 881.1 | 21.2 | 25.1 | 787.2 | 3.7 | | -17.5 | -94.0 | Yes |
| DG7-7 | 26.3 | 888.6 | 21.8 | 25.4 | 852.6 | 20.0 | | -1.8 | -36.0 | |
| DG8-1 | 24.7 | 780.2 | 20.3 | 23.6 | 995.8 | 1.7 | | -18.6 | 215.6 | Yes |
| DG8-2 | 23.1 | 768.7 | 21.2 | 22.8 | 944.5 | 1.7 | | -19.5 | 175.8 | Yes |
| DG8-3 | 22.9 | 771.5 | 21.4 | 23.2 | 811.0 | 2.1 | | -19.2 | 39.5 | Yes |
| DGB-4 | 22.9 | 764.4 | 20.4 | 22.9 | 831.7 | 1.9 | | -18.5 | 67.4 | Yes |
| DG8-5 | 23.8 | 807.3 | 19.4 | 24.1 | 850.7 | 1.0 | | -18.4 | 43.4 | Yes |
| DG8-6 | 23.4 | 809.6 | 19.9 | 22.2 | 784.4 | 2.3 | | -17.6 | -25.2 | Yes |
| DG8-7 | 23.9 | 803.0 | 19.9 | 24.4 | 768.4 | 17.0 | | -2.9 | -34.6 | |
| DG9-1 | 27.0 | 970.4 | 18.5 | 26.9 | 955.3 | 20.2 | | 1.7 | -15.2 | |
| DG9-2 | 27.9 | 800.6 | 20.8 | 26.2 | 801.9 | 20.7 | | -0.2 | 1.2 | |
| DG9-3 | 26.9 | 799.2 | 21.1 | 25.6 | 776.6 | 21.2 | | 0.0 | -22.6 | |
| DG9-4 | 26.3 | 784.5 | 21.0 | 25.4 | 772.1 | 21.0 | | 0.0 | -12.4 | |
| DG9-5 | 27.7 | 792.7 | 20.4 | 25.5 | 781.4 | 20.3 | | -0.1 | -11.3 | |

### Discussion).

### Discussion and Conclusions

The headspace oxygen concentration and total pressure were successfully measured for all syringes of each of the nine configurations (Table 1). The headspace oxygen concentration measurement uncertainty for this study was established as ±0.8% atm based upon the oxygen standards; the corresponding measurement uncertainty associated with the total pressure was established as the greater of ±6 torr and ±7% of the measured value based upon the performance of the pressure standards. Because the syringes contained liquid water, the headspace was saturated with water vapor and, thus, provided the maximum FMS signal that could be obtained for the described syringe configurations.

The results of the measurements are summarized in Table 3. The change in the headspace oxygen concentration [%] and total pressure [torr] observed after the cyrogenic storage is also tabulated. Under these conditions, a breach in container closure integrity in a particular syringe may decrease the oxygen concentration in its headspace via three possible processes. First, a decrease may occur because the nitrogen vapor surrounding the syringes in the cryo-storage container will leak into the syringe through a breach (defect) as the temperature decreases, this may result in a decrease in oxygen concentration due to dilution of total oxygen in the headspace. Second, after the syringes thermally equilibrate at the ~177°C storage temperature, diffusion may occur such that oxygen molecules will leak out of the syringe headspace and into the nitrogen environment of the cryo-storage container. Third, once the syringes are removed from cryo-storage, the temperature increase may generate an over-pressure in the headspace of any syringe that had developed a leak; this over-pressure will tend to push out the syringe headspace gas, including any oxygen that still remains. Note that if the breach in container closure integrity is re-sealed as the temperature increases, the headspace over-pressure will be maintained. The "Plunger Failure" column in Table 3 indicates where the presence of over-pressure in a syringe headspace pushed the syringe plunger up.

Based upon the change of oxygen content observed for the negative controls (Sample Nos. 7), a decrease greater than 3.0% in the headspace oxygen concentration was scored as indicating container closure failure. Furthermore, any syringe for which the plunger was pushed out after the syringe warmed up ("Plunger Failure") was also considered a container closure failure. Finally, a headspace pressure increase of greater than 100 torr was treated as an additional criterion that a CCI failure had occurred.

Displayed in Figure 4 and tabulated in Table 3, the results for the individual syringes indicate that 29 out of the 45 syringe samples that were stored at the cyrogenic temperature lost container closure. As expected, all eight of the positive controls (Sample Nos. 6) also failed.

All five replicates for Sample Formats 1, 4, 5, and 8 lost CCI, as well as four out of the five test syringes for Sample Format 3. Each of these formats used plungers, alone, to seal the barrel of the syringe. Interestingly, Sample format 8 included two plungers. Sample Format 7, which included a stopper at the barrel end was the most successful at preventing failure.
The invention will now be described by way of a series of numbered clauses.
1. A pharmaceutical composition for maintaining viability of a therapeutic mammalian cell comprising:
   a freezing medium;
   a freezing agent; and
   an organic polymer.
2. The pharmaceutical composition of clause 1, wherein the agent includes dimethyl sulfoxide.
3. The pharmaceutical composition of any of clauses 1-2, wherein the medium excludes animal serum.
4. The pharmaceutical composition of any of clauses 1-3, wherein the polymer is a hyaluronic acid.
5. The pharmaceutical composition of any of clauses 1-4, wherein the cells are derived from disc tissue, skin, muscle, intestine, bone marrow, neural, liver, heart, lung, pancreas, articular cartilage, bone, thymus, thyroid, or lymph tissue.
6. The pharmaceutical composition of any of clauses 1-5, wherein the cells are derived from disc tissue, articular cartilage, heart tissue, or bone.
7. A device for freezing and thawing a therapeutic cell mixture, comprising:
   a container comprising;
      a first open end having a first diameter;
      a second open end having a second diameter that is smaller than the first diameter;
      a lumen, comprising a lumen wall, in fluid communication with the first and second open end, and having a lumen diameter that is similar to the first diameter, wherein the container is constructed of one or more biocompatible materials that maintain structural integrity when frozen at less than about -130 °C and thawed;
   a first polymeric seal configured to seat within the lumen and create a sealing contact with the lumen wall;
   a first polymeric cap designed to seal the first open end; and
   a second polymeric cap designed to seal the second open end.
8. The container of clause 7, wherein the container defines a syringe barrel, and the first polymeric seal is a piston or plunger.
9. The container of any of clauses 7-8, wherein the container is made of a translucent polymer.
10. The container of any of clauses 7-9, wherein the container is made of a polymeric cyclic olefin.
11. The container of any of clauses 7-10, wherein the second open end defines a luer lock able to accept a syringe needle with a compatible structure when the second polymeric cap is removed.
12. A method of freezing a therapeutic mammalian cell, comprising:
   combining a plurality of therapeutic cells and a composition to create a mixture;
   placing the mixture into a container of a medical device having a first opening and a second opening;
   positioning a first polymeric seal in contact with the mixture;
   placing a first polymeric cap at the first opening and a second polymeric cap at the second opening to create a gas tight seal;
   reducing the temperature of the mixture to -80 °C or less;
   maintaining the mixture at a temperature of less than about -80 °C for at least 30 days, wherein after 30 days at least about 50% of the cells grow and divide at least once in cell culture.
13. The method of clause 12, wherein the mixture is maintained in liquid nitrogen vapor phase and less than about -130 °C or at least part of the 30 days.
14. The method of any of clauses 12-13, wherein at least about 90% of the cells grow and divide at least once after 30 days.
15. The method of any of clauses 12-14, wherein after maintaining the cells, the mixture is thawed to liquid form having a temperature between about 20 and 40 °C.
16. The method of any of clauses 12-15, wherein the therapeutic cells are derived from human intervertebral disc tissue.
17. The method of any of clauses 12-16, wherein the container comprises
   a first open end with a first diameter;
   a second open end with a second diameter;
   a lumen, comprising a lumen wall, in fluid communication with the first and second open end, wherein the container is constructed of one or more biocompatible materials that maintain structural integrity when frozen at less than about -130 °C and thawed.
18. The method of any of clauses 12-17, wherein the device prevents exchange of at least nitrogen gas when the device is stored at temperatures below -80°C.
19. The method of any of clauses 12-18, wherein the cells are derived from disc tissue, skin, muscle, intestine, bone marrow, neural, liver, heart, lung, pancreas, articular cartilage, bone, thymus, thyroid, or lymph tissue.
20. The pharmaceutical composition of any of clauses 12-19, wherein the cells are derived from disc tissue, articular cartilage, heart tissue, or bone.

## Claims

1. A method of freezing a therapeutic mammalian cell, comprising:
combining a plurality of therapeutic cells and a composition to create a mixture;
placing the mixture into a container of a medical device having
a first opening and a second opening;
positioning a first polymeric seal in contact with the mixture;
placing a first polymeric cap at the first opening and a second polymeric cap at the second opening to create a gas tight seal;
reducing the temperature of the mixture to -80 °C or less;
maintaining the mixture at a temperature of less than about -80 °C for at least 30 days, wherein after 30 days at least about 50% of the cells grow and divide at least once in cell culture.

2. The method of claim 1, wherein the mixture is maintained in liquid nitrogen vapor phase and less than about -130 °C for at least part of the 30 days.

3. The method of claim 1 or 2, wherein at least about 90% of the cells grow and divide at least once after 30 days.

4. The method of any of claims 1-3, wherein after maintaining the cells, the mixture is thawed to liquid form having a temperature between about 20 and 40 °C.

5. The method of any of claims 1-4, wherein the container comprises
a first open end with a first diameter;
a second open end with a second diameter;
a lumen, comprising a lumen wall, in fluid communication with the first and second open end, wherein the container is constructed of one or more biocompatible materials that maintain structural integrity when frozen at less than about -130 °C and thawed.

6. The method of any of claims 1-5, wherein the device prevents exchange of at least nitrogen gas when the device is stored at temperatures below -80°C.

7. The method of any of claims 1-6, wherein the cells are derived from disc tissue, skin, muscle, intestine, bone marrow, neural, liver, heart, lung, pancreas, articular cartilage, bone, thymus, thyroid, or lymph tissue.

8. The method of any of claims 1-7, wherein the cells are derived from disc tissue, articular cartilage, heart tissue, or bone.

9. The method of any of claims 1-8, wherein the therapeutic cells are derived from human intervertebral disc tissue.
